# EUROPEAN PATENT APPLICATION

(11) **EP 1 130 119 A1**
(43) Date of publication of application: **05.09.2001**
(21) Application number: 01200532.8
(22) Date of filing: 15.02.2001
(51) Int. Cl.: C12Q 1/68

(54) **Reducing background in hybridisation reactions**

(30) Priority: 17.02.2000 EP 00200549
(71) Applicant: Amsterdam Support Diagnostics B.V., 1105 BA Amsterdam (NL)
(72) Inventor: de Baar, Marinus Petrus, 3582 XP Utrecht (NL); van Gemen, Bob, 1326 HV Almere (NL); Timmermans, Eveline Catherina Anna Clasina, 5231 HS 's-Hertogenbosch (NL)
(74) Representative: Prins, Adrianus Willem

(57) **Abstract**

The invention provides a method for reducing background in hybridisation reactions of nucleic acids involving at least two homologous probes, wherein at least one of said probes is non-linear, or two homologous target sequences and a non-linear probe. Background is reduced by introducing an intended mismatch with a target sequence in at least one of said probes. The presence of the mismatch reduces the specificity of probes not entirely complementary to a target sequence to such an extent that the background signal is reduced. A set of mixed homologous probes, wherein at least one of said probes is non-linear, comprising such specific mismatch is also provided. Said set can be used for the detection of variants of a family of nucleic acids, for instance a number of HIV variants. The invention also provides kits for carrying out the methods according to the invention.

## Description

### Introduction and field of invention.

The present invention relates to the field of molecular biology. In particular the invention relates to methods for detecting, identifying and/or distinguishing between nucleic acid molecules or functional analogues thereof, such as PNA's.

The most common method for identification of a nucleic acid sequence is the hybridization of a sequence specific short piece of DNA (probe) to the complementary sequence in the target nucleic acid (DNA or RNA), possibly followed by extension of the probe through the action of a nucleic acid polymerase or ligase. Usually the probe is labeled (directly, indirectly; before, during or after hybridisation) with a detectable moiety, for instance a radioactive or fluorescent group, indicating the presence of the (hybridised) probe at a certain position or place. In a typical protocol the probe-target complex that is formed after the hybridization is washed (bound-free separation) to remove non-bound probe. The amount of probe that remains attached to the target as indicated by the label is a measure for the amount of target that has a complementary sequence of the probe. In case no signal is obtained the target sequence was absent, or at least below detection levels.

Next to wide spread application in the field of molecular biology, this method of probe hybridization is also commonly used for the detection and quantification of nucleic acid belonging to pathogenic microorganisms in clinical samples. In some protocols the nucleic acid from the microorganism is first amplified with a nucleic acid amplification method like PCR, NASBA, SDA, TMA or others, before the amplified nucleic acid is detected by probe hybridization. In more recently described methods the probe hybridization takes place during the generation of the amplified nucleic acid in the amplification reaction itself. In this protocol the signal of the label attached to the probe only becomes detectable after the probe has hybridized to the complementary nucleic acid. Examples of such probes that enable real-time homogeneous detection in amplification reactions are the TaqMan^{1, 2} and Molecular Beacon^{3, 4} probes.

Another feature of probes is the identification of small changes (i.e. mutations) in the nucleotide sequence. Single nucleotide mutations and larger mutations, including insertions and deletions, can be detected by the application of specific probes that are the complement of the sequence encompassing the mutation. Commonly the probes are short oligonucleotides consisting of approximately 15-50, preferably about 20 nucleotides with a mutated position somewhere in the middle of the sequence. In case that there is no complete match between the probe and the target sequence the probe will not hybridize or hybridize with reduced efficiency. Only the completely matched probe will give a good detectable signal, and if multiple probes are used that are specific for different sequences with mutations the probe that in the end gives the signal matched the target and the mutation is identified. There are many variations on this theme, but the basic principle of two complementary sequences that hybridize when there are no mismatches is always present. This strategy for identification of single nucleotide mutations is preferably applied to molecular beacon probes^{6, 7}, because these non-linear probes have a high specificity.

A problem however, when looking for small variations in target sequences, such as point mutations, is that when mixed probes are applied, those probes that have only a mismatch at the site of the point mutation will hybridise to the target sequence, competing with the probe that has an exact complementary sequence to the target sequence. Although this binding is weaker than that of the exact fit, it gives rise to a background, which may be considered a positive signal and may therefor even lead to false positives. The reverse is also true. When there are homologous target sequences present competition for a single kind of probe may occur. Even in systems where single probes and/or single target sequences per container are used, when there are large homologies in hybridising areas which are the same in different containers containing related, but not identical probes and/or target sequences, the results start to overlap and the distinguishing capacity may be insufficient.

### Summary of the invention

We found that the introduction of a mismatch in a non-linear probe, such as a beacon probe, enhances the specificity of the probe in a mixed set of homologous probes for the detection of point mutations in a sequence. We also found that using a single non-linear probe having a mismatch for at least one of a member of a family of target sequences also enhances the specificity by reducing background signals. This result is unexpected, because until the present invention it was stated that introduced mismatches in non-linear probes resulted in very unstable hybrids⁷. It was suggested that a hairpin probe, such as a beacon probe, hardly binds its target sequence anymore after one introduced mismatch. Only linear probes would significantly bind their target sequence after the introduction of a mismatch. Therefore, only linear probes were thought to be suitable for intended introduction of a mismatch to reduce background. However, we have found that hybridisation of non-linear probes comprising a mismatch with a target sequence is indeed possible, and that the amount of formed hybrids and the stability of said hybrids is sufficient to perform identification of a nucleic acid sequence. Moreover, the introduction of an intended mismatch in non-linear probes reduces background in hybridisation reactions.

Thus the invention provides a method for reducing background in a hybridisation reaction of nucleic acids involving mixed homologous probes, wherein at least one of said probes is non-linear, comprising introducing a mismatch with an intended target sequence in at least one of the non-linear probes. The presence of the mismatch reduces the specificity of probes not entirely complementary to a target sequence to such an extent that the background signal is at least significantly reduced. This is particularly useful in methods where the probes are very similar, for instance when single point mutations must be detectable. Thus in a preferred method the invention provides a method in which the probes are designed to detect point mutations in target sequences, more specifically a method wherein at least two of said probes comprise an identical sequence except for the variation of the point mutation and possibly the site of the mismatch. This does not mean that the sequences must be identical over the whole of the molecule, but that they are identical in the part where hybridisation should occur. This is a situation in which false positives are a significant risk. The mismatch should comprise as many nucleotides as necessary to significantly lower the background, but not so many nucleotides that the probe having the exact match for the allelic variation (point mutation) has a significantly lower binding affinity. The number depends of course on the length of the probe and the base composition of the probe. Typically no more than 10 percent of the probe should be mismatch, preferably less than 5%, and especially about 1-3 nucleotides in a 20 nucleotide probe or the corresponding percentage in a shorter or longer probe. Thus in a further embodiment the invention provides a method wherein the mismatch comprises 1-3 nucleotides. For the same reasons as mentioned above the mismatch should be located not too close, but also not too far away from the actual site of variation. Typically in a 20 nucleotide probe it should be located between 2 and 5 nucleotides from the site of variation. Thus in a further embodiment the invention provides a method wherein said mismatch is located between 2 and 20 nucleotides up-or downstream of said point mutation.

Probe length is not really critical. Conventional probe lengths are suitable. Usually probes should not exceed 50 and should not be less than 15 nucleotides, with a good average at about 20. Thus in yet another embodiment the invention provides a method wherein at least one non-linear probe has a length of about 15-50 nucleotides.

As stated herein before a label is typically applied for detection of bound (sometimes unbound) probe. The label may be any conventional label and it may be attached to the probe or the hybridised complex at any suitable time. Thus in yet another embodiment the invention provides a method wherein at least one of said mixed homologous non-linear probes is provided with a detectable moiety. Before or after the hybridisation step conventional amplification and/or purification steps may be employed in the methods of the invention. All such methods are well known in the art and need no further explanation here.

Thus the invention further provides a method which includes an amplification step.

Sets of probes designed for the methods of the present invention are also provided by the invention. Thus the invention provides e.g. a set of mixed homologous probes for detection of at least one allelic variant of a nucleic acid family, wherein at least one of said probes is non-linear, said probes comprising sequences completely complementary to and specific for one of the allelic variants of said family, except for a specific mismatch located upstream and/or downstream from the site of variation.

The invention further provides such a set of mixed homologous primers, wherein at least two of said probes comprise an identical sequence except for the variation of a point mutation and possibly the site of the mismatch, preferably a set wherein said mismatch comprises 1-3 nucleotides. The reasons for the design of the sets of primers have been explained above and will become more apparent from the experimental part.

The invention also provides such a set wherein said mismatch is located 2-20 nucleotides upstream or downstream of said point mutation, whereby the probes typically have lengths between 15 and 50 nucleotides.

The invention also provides the use of the methods and the probes in molecular biology in general and in the detection of point mutations and allelic variants in particular, especially in the field of detection of pathogens, in particular of HIV variants. Thus the invention further provides the use of a set of probes according to the invention for the detection of variants of a family of nucleic acids, particularly wherein the family of nucleic acids is derived from a family of pathogens, in particular, wherein said family represents a number of HIV-variants. Kits for carrying out the methods according to the invention are also provided.

The invention is further explained by the use of the following example. This illustrative example does not limit the invention in any way.

### Example 1

In this example nucleic acid extracted from the supernatant of HIV-1 *in vitro* cultures was amplified with NASBA using different primer sets for HIV-1 RNA (gag region) amplification. The HIV-1 viruses used in this example were of the subtypes A, B and C, which could be distinguished by mutations in the gag region that was amplified. The nucleic acid was extracted and purified using the "Boom" method (Boom R, Sol CJ, Salimans MM, Jansen CL, Wertheim-van Dillen PM, van der Noordaa J, 1990. Rapid and simple method for purification of nucleic acids. *J Clin Microbiol;* 28(3):495-503). After the extraction nucleic acid was eluted in 50µl buffer (10 mM tris, pH7.5, 1 mM EDTA) or water and stored at -20°C. For amplification by NASBA 5 µl of this nucleic acid solution was used as input for the amplification reactions. The primers and molecular beacon probes (For reference see: Leone G, van Schijndel H, van Gemen B, Kramer FR, Schoen CD [1998] Molecular beacon probes combined with amplification by NASBA enable homogeneous, real-time detection of RNA. *Nucleic Acids Res* May 1;26(9):2150-2155) that were used in the experiment are described in table 1.

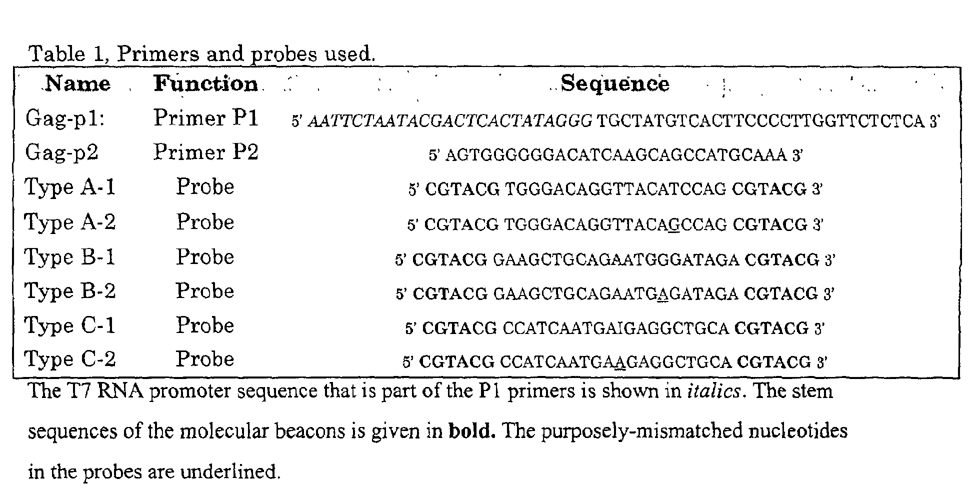

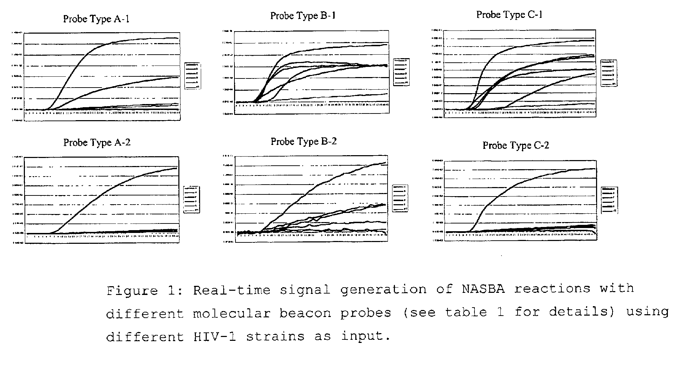

The molecular beacon probes that are used in this experiment are labeled with TET, ROX or FAM (the label) at the 5' ends for respectively type A, type B and type C. All probes are labeled with DABCYL (the quencher) at the 3' end. The NASBA reactions (Tris-HCl 40 mM, pH=8.5, MgCl₂ 12 mM, KCl 70 mM, DTT 5 mM, dNTP's (each) 1 mM, rATP 2 mM, rUTP 2 mM, rCTP 2 mM, rGTP 1.5 mM, ITP 0.5 mM, EDTA 0.75 mM, DMSO 15% v/v, oligonucleotide P1 0.2 µM, oligonucleotide P2 0.2 µM, molecular beacon probe 0.2 µM and Sorbitol 0.375 M) were incubated at 65°C for 5 minutes and subsequently at 41°C for 5 minutes. Than the enzyme mix was added (BSA 2.1 mg, RNase H 0.01 units, T7 RNA Polymerase 37 units, AMV-RT 7.5 units) and after gentle mixing by tapping the reactions were incubated at 41°C in a fluorimeter (Cytofluor 4000, Perkin Elmer or ABI 7700, ABI) for 90 minutes with measurement of the fluorescent signal every minute. The results of the experiment are shown in figure 1.

From the results as shown in figure 1 it is clear that the introduction of a purposely made mismatch has resulted in increased specificity of the probes (compare lower three panel with the upper three panels in figure 1).

### Brief description of the drawings

Figure 1: Real time signal generation of NASBA reactions with different molecular beacon probes (see table 1 for details) using different HIV-1 strains as input.

### References

1. Morris T, Robertson B, Gallagher M.
   Rapid reverse transcription-PCR detection of hepatitis C virus RNA in serum by using the TaqMan fluorogenic detection system.
   J Clin Microbiol. 1996 Dec;34(12):2933-6.
2. Heid CA, Stevens J, Livak KJ, Williams PM.
   Real time quantitative PCR.
   Genome Res. 1996 Oct;6(10):986-94.
3. Tyagi S, Kramer FR.
   Molecular beacons: probes that fluoresce upon hybridization.
   Nat Biotechnol. 1996 Mar;14(3):303-8.
4. Leone G, van Schijndel H, van Gemen B, Kramer FR, Schoen CD.
   Molecular beacon probes combined with amplification by
   NASBA enable homogeneous, real-time detection of RNA.
   Nucleic Acids Res. 1998 May 1;26(9):2150-5
5. Holloway JW, Beghe B, Turner S, Hinks LJ, Day IN, Howell WM.
   Comparison of three methods for single nucleotide polymorphism typing for DNA bank studies: sequence-specific oligonucleotide probe hybridisation, TaqMan liquid phase hybridisation, and microplate array diagonal gel electrophoresis (MADGE).
   Hum Mutat. 1999;14(4):340-7.
6. Marras SA, Kramer FR, Tyagi S.
   Multiplex detection of single-nucleotide variations using molecular beacons.
   Genet Anal. 1999 Feb;14(5-6):151-6.
7. Tyagi S, Bratu DP, Kramer FR.
   Multicolor molecular beacons for allele discrimination.
   Nat Biotechnol. 1998 Jan;16(1):49-53.

## Claims

1. A method for reducing background in hybridisation reactions of nucleic acids involving at least two homologous probes, wherein at least one of said probes is non-linear, comprising introducing a mismatch with an intended target sequence in at least one of the non-linear probes.

2. A method for reducing background in hybridisation reactions of nucleic acids involving at least two homologous target sequences, comprising providing for an intended mismatch between at least one of the target sequences and at least one non-linear probe for hybridisation.

3. A method according to claim 1 or 2 in which the probes are designed to detect point mutations in at least one target sequence.

4. A method according to claim 2, wherein at least two of said probes and/or two of said target sequences comprise an identical sequence except for the variation of the point mutation and possibly the site of the mismatch.

5. A method according to any one of claims 1-4, wherein the mismatch comprises 1-3 nucleotides.

6. A method according to any one of claims 2-5, wherein said mismatch is located between 2 and 20 nucleotides up-or downstream of said point mutation.

7. A method according to any one of claims 1-6 wherein at least one non-linear probe has a length of about 15-50 nucleotides.

8. A method according to any one of claims 1-7 wherein at least one of the non-linear probes is provided with a detectable moiety.

9. A method according to any one of claims 1-8, which includes an amplification step.

10. A set of mixed homologous probes for detection of at least one allelic variant of a nucleic acid family, wherein at least one of said probes is non-linear, said probes comprising sequences completely complementary to and specific for one of the allelic variants of said family, except for a specific mismatch located upstream and/or downstream from the site of variation.

11. A set of mixed homologous primers according to claim 10, wherein at least two of said probes comprise an identical sequence except for the variation of a point mutation and possibly the site of the mismatch.

12. A set according to claim 11, wherein said mismatch comprises 1-3 nucleotides.

13. A set according to claim 11 or 12 wherein said mismatch is located 2-20 nucleotides upstream or downstream of said point mutation.

14. A set according to any one of claims 10-13 wherein the probes have lengths between 15 and 50 nucleotides.

15. A set according to any one of claims 11-14 wherein said mixed homologous probes are in a single container.

16. Use of a set of probes according to any one of claims 10-14 for the detection of variants of a family of nucleic acids.

17. Use according to claim 16 wherein the family of nucleic acids is derived from a family of pathogens.

18. Use according to claim 17 wherein said family represents a number of HIV-variants.

19. A kit for the detection of at least one target sequence from a family of target sequences, comprising at least one non-linear probe complementary to a target sequence specific for said family of target sequences and having a mismatch in said complementarity for at least one of the target sequences from said family and further comprising suitable means for detection and/or amplification and/or isolation of nucleic acids.

20. A kit according to claim 19, comprising a set of mixed homologous probes according to any one of claims 11-15.


### Annex to the application documents - subsequently filed sequences listing

SEQUENCE LISTING

<110> Amsterdam Support Diagnostics B.V.

<120> Reducing background in hybridisation reactions

<130> P52531EP10

<140> 01200532.8
<141> 2001-02-15

<150> EP00200549.4
<151> 2000-02-17

<160> 8

<170> PatentIn Ver. 2.1

<210> 1
<211> 54
<212> DNA
<213> Artificial Sequence

<220>
<221> misc_feature
<222> (1)..(54)

<220>
<223> Description of Artificial Sequence: Primer P1 Gag-p1

<400> 1
aattctaata cgactcacta tagggtgcta tgtcacttcc ccttggttct ctca        54

<210> 2
<211> 29
<212> DNA
<213> Artificial Sequence

<220>
<223> Description of Artificial Sequence:Primer P2 Gag-p2

<220>
<221> misc_feature
<222> (1)..(29)

<400> 2
agtgggggga catcaagcag ccatgcaaa                                    29

<210> 3
<211> 31
<212> DNA

<213> Artificial Sequence

<220>
<223> Description of Artificial Sequence: Probe Type A-1

<220>
<221> misc_feature
<222> (1)..(31)

<400> 3
cgtacgtggg acaggttaca tccagcgtac g                                    31



<210> 4
<211> 31
<212> DNA
<213> Artificial Sequence

<220>
<223> Description of Artificial Sequence: Probe Type A-2

<220>
<221> misc_feature
<222> (1)..(31)

<400> 4
cgtacgtggg acaggttaca gccagcgtac g                                    31



<210> 5
<211> 33
<212> DNA
<213> Artificial Sequence

<220>
<221> misc_feature
<222> (1)..(33)

<220>
<223> Description of Artificial Sequence: Probe Type B-1

<400> 5
cgtacggaag ctgcagaatg ggatagacgt acg                                  33



<210> 6
<211> 33
<212> DNA
<213> Artificial Sequence

<220>
<223> Description of Artificial Sequence: Probe Type B-2

<220>
<221> misc_feature
<222> (1)..(33)

<400> 6
cgtacggaag ctgcagaatg agatagacgt acg                                33

<210> 7
<211> 32
<212> DNA
<213> Artificial Sequence

<220>
<223> Description of Artificial Sequence: Probe Type C-1

<220>
<221> misc_feature
<222> (1)..(32)
<223> /note="N stands for inosine"

<400> 7
cgtacgccat caatgangag gctgcacgta cg                                 32

<210> 8
<211> 32
<212> DNA
<213> Artificial Sequence

<220>
<223> Description of Artificial Sequence: Probe Type C-2

<220>
<221> misc_feature
<222> (1)..(32)

<400> 8
cgtacgccat caatgaagag gctgcacgta cg                                 32